# EUROPÄISCHE PATENTSCHRIFT

(11) **EP 1 526 848 B1**
(45) Veröffentlichungstag und Bekanntmachung des Hinweises auf die Patenterteilung: **18.07.2007**
(21) Anmeldenummer: 03775130.2
(22) Anmeldetag: 08.08.2003
(51) Int. Cl.: A61K 9/70

(54) **OPIOD-REZEPTOR-ANTAGONISTEN IN TRANSDERMALEN SYSTEMEN MIT BUPRENORPHIN**
OPIOID-RECEPTOR ANTAGONISTS IN TRANSDERMAL SYSTEMS HAVING BUPRENORPHINE
ANTAGONISTES DU RECEPTEUR OPIOIDE DANS DES SYSTEMES TRANSDERMIQUES A BASE DE BUPRENORPHINE

(30) Priorität: 09.08.2002 DE 10237056; 09.08.2002 DE 10237057
(43) Veröffentlichungstag der Anmeldung: 04.05.2005
(73) Patentinhaber: Grünenthal GmbH, 52078 Aachen (DE)
(72) Erfinder: BARTHOLOMÄUS, Johannes, 52080 Aachen (DE); KUGELMANN, Heinrich, 52068 Aachen (DE); PAQUES, Eric-Paul, 52076 Aachen (DE)
(86) Internationale Anmeldenummer: PCT/EP2003/008828
(87) Internationale Veröffentlichungsnummer: WO 2004/014336

(56) Entgegenhaltungen:
- EP-A- 0 430 019
- EP-A- 0 819 438
- WO-A-97/33566
- WO-A-98/36728
- US-A- 5 900 420
- HAYES A G ET AL: "EVALUATION OF THE RECEPTOR SELECTIVITIES OF OPIOID DRUGS BY INVESTIGATING THE BLOCK OF THEIR EFFECT ON URINE OUTPUT BY BETA-FUNALTREXAMINE" JOURNAL OF PHARMACOLOGY AND EXPERIMENTAL THERAPEUTICS, AMERICAN SOCIETY FOR PHARMACOLOGY AND, US, Bd. 240, Nr. 3, 1987, Seiten 984-988, XP001064229 ISSN: 0022-3565
- LEANDER J D: "BUPRENORPHINE HAS POTENT KAPPA OPIOID RECEPTOR ANTAGONIST ACTIVITY" NEUROPHARMACOLOGY, PERGAMON PRESS, OXFORD, GB, Bd. 26, Nr. 9, 1987, Seiten 1445-1447, XP001064223 ISSN: 0028-3908
- MARK L. ET. AL.: "Buprenorphine is an Antagonist at the k Opioid Receptor" PHARMACEUTICAL RESEARCH, 1985, Seiten 178-181, XP009029866
- BORKOWSKI K. : "k-Opioid agonist induced diuresis." CAN. J. PHYSIOL. PHARMACOL. , Bd. 67, 1989, Seiten 1219-1224, XP009029870

## Beschreibung

Die Erfindung betrifft Opiod-Rezeptor-Antagonisten, wie beispielsweise Naloxon, in transdermalen Applikationsformen mit mindestens Buprenorphin als Wirkstoff.

Betäubungsmittelhaltige und insbesondere opioidhaltige Produkte beinhalten grundsätzlich das Risiko des Missbrauchs, wobei die Höhe dieses Missbrauchsrisikos im allgemeinen davon abhängt, mit welchem Aufwand der potentiell Missbrauchende den erwünschten euphorischen Effekt erzielen kann. Aufgrund der schlechten oralen Verfügbarkeit der meisten Opioide ist meist Voraussetzung zur Erzielung eines solchen euphorischen Effekts die parenterale Applikation einer opiodhaltigen Lösung. Im Falle eines Missbrauchs wird versucht, eine parenteral applizierbare Arzneiform zu gewinnen, auch wenn das hierzu missbrauchte Medikament in einer dafür primär nicht geeigneten Arzneiform vorliegt. Am einfachsten ist der Mißbrauch natürlich bei Lösungen oder Arzneiformen, die vollständig aufgelöst werden können. Ordnungsgemäß hergestellte parenterale Lösungen sind klar, isoton, isohydrisch, steril, pyrogenfrei und enthalten keine ungelösten Bestandteile. Alle diese Anforderungen zu erfüllen, ist bei missbräuchlicher Herstellung praktisch nicht möglich; das damit zusätzlich vorhandene Gesundheitsrisiko wird aber in Kauf genommen. Transdermale Systeme, die im Fokus dieser Erfindung stehen, sind von der Konstruktion eher ungeeignet für Missbrauchszwecke. Trotzdem ist natürlich prinzipiell bei opioidhaltigen Pflastern aufgrund der Attraktivität des Wirkstoffs potentiell ein Risiko des Missbrauchs nicht auszuschliessen. In diesem Fall müßte dann das Betäubungsmittel aus der Matrix herausgelöst werden, wobei - sofern dies überhaupt gelingt - die zwangsläufig zusätzlich mit eluierten Hilfsstoffe die Lösung weiter verunreinigen, und somit eine so gewonnene parenterale Applikationsform unattraktiv ist.

Damit erscheinen grundsätzlich transdermale Systeme für den Mißbrauch ungeeignet. Entsprechend gibt es auch im Stand der Technik bisher keine weiteren Missbrauchshemmnisse in Transdermalen Systemen. Allerdings sollte rein vorsorglich einkalkuliert werden, dass potentiell Mißbrauchende Möglichkeiten entdecken könnten, die dem transdermalen System inherenten Schwierigkeiten für den Mißbrauch zu überwinden.

Entsprechend war es Aufgabe der vorliegenden Erfindung, eine Möglichkeit zu finden, das Mißbrauchsrisiko bei opiodhaltigen, insbesondere buprenorphinhaltigen, Transdermalen Systemen weiter zu senken. Entsprechend ist Erfindungsgegenstand ein Transdermales System bzw. Trandermales Therapeutisches System enthaltend als Wirkstoff mindestens Buprenorphin in Form der Base oder eines Salzes, dadurch gekennzeichnet, dass es mindestens einen µ, κ oder δ Opioidrezeptor-Antagonisten; gegebenenfalls in Form seiner Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form der Säuren oder Basen oder in Form der Salze, insbesondere der physiologisch verträglichen Salze, oder in Form der Solvate, insbesondere der Hydrate; enthält.

Es wurde nun herausgefunden, daß der Missbrauch so zusätzlich erschwert bis unmöglich gemacht wird. Dem Transdermalen System wird gleichzeitig ein parenteral wirksamer Opioid-Antagonist mit zugesetzt oder das buprenorphinhaltige Pflaster mit einer Opioid-Antagonist-Lösung zusätzlich beschichtet. Dadurch wird beim Versuch, das Buprenorphin aus dem Pflaster herauszulösen, der Antagonist ebenfalls mit herausgelöst. Bei der parenteralen Applikation einer solchen Lösung besetzt der Antagonist aufgrund seiner großen Affinität, beispielsweise zum µ-Opioid-Rezeptor sofort die Rezeptoren und verhindert dadurch die euphorische Wirkung, da eine Rezeptorbindung des Buprenorphins als Agonist dann nicht mehr möglich ist.

Unter dem Begriff Salz ist generell jegliche Form eines erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind.

Unter dem Begriff des (physiologisch verträglichen) Salzes, insbesondere mit Säuren versteht man im Sinne dieser Erfindung Salze mindestens einer der erfindungsgemäßen Verbindungen - meist, beispielsweise an einem Stickstoff, protoniert - als Kation mit mindestens einem Anion, die physiologisch - insbesondere bei Anwendung im oder am Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, α-Liponsäure, Acetylglycin, Acetylsalicylsäure, Hippursäure und/oder Asparaginsäure. Besonders bevorzugt ist das Hydrochlorid-, Hydrobromid-Salz und das Hydrogencitrat.

Besonders bevorzugt ist es, wenn der im erfindungsgemäßen Transdermalen System enthaltene µ, κ oder δ Opioidrezeptor-Antagonist ein µ Opioidrezeptor-Antagonist bzw. Morphinantagonist ist, vorzugsweise Levallorphan, Naltrexon, Nalorphin oder Naloxon, insbesondere Naloxon.

Besonders bevorzugt ist diese Ausführungsform, wenn das im erfindungsgemäßen Arzneimittel enthaltene Levallorphan, Naltrexon, Nalorphin oder Naloxon in Form seiner Base oder in Form der Salze, insbesondere der physiologisch verträglichen Salze, vorzugsweise der mit anorganischen oder organischen Säuren gebildeten Salze, insbesondere als Chlorid- Bromid- oder Hydrogencitratsalz; vorliegt.

Besonders bevorzugt ist es, wenn das im erfindungsgemäßen Transdermalen System enthaltene Buprenorphin als Base vorliegt.

Unter einem transdermalen therapeutischen System (TTS) bzw. Transdermalen System soll nach Zafaroni "eine arzneistoffenthaltende Vorrichtung bzw. eine Darreichungsform, die einen Arzneistoff oder mehrere in vorausbestimmter Rate kontinuierlich über einen festgesetzten Zeitraum an einen festgelegten Anwendungsort abgibt" (zitiert nach Heilmann, therapeutische Systeme - Konzept und Realisation programmierter Arzneiverabreichung, 4. Auflage, Ferdinand Enke-Verlag Stuttgart 1984, Seite 26) verstanden werden, wobei im vorliegenden Fall der Anwendungsort die Haut ist.

Der Aufbau von transdermalen Systemen ist dem Fachmann bekannt. Schutzrechte, in denen der grundsätzliche Aufbau beschrieben wird, sind beispielsweise DE 3315272, DE 3843239, US 3,598,122. Der Inhalt dieser Patentschriften und Anmeldungen ist vollinhaltlich Teil dieser Beschreibung.

Wird ein transdermales therapeutisches System auf die Haut eines Patienten appliziert, soll der Arzneistoff abgegeben werden, um beim Patienten topisch oder systemisch wirksam zu werden. Arzneiformen dieser Art werden bereits therapeutisch genutzt. Sie sind meist schichtförmig aufgebaut und bestehen im einfachsten Falle aus einer Rückschicht, einem selbstklebenden Wirkstoffreservoir und einer wieder ablösbaren Schutzschicht, die vor der Applikation zu entfernen ist:

Besonders bevorzugt sind Transdermale Systeme wie sie in der WO98/36728, der WO 96/19975 bzw. der US 6,264,980 oder der EP 430 019 A2 bzw. CA 2030178 beschrieben sind, wobei der gesamte Inhalt dieser Schriften sowie der Inhalt der dort zitierten Literatur Teil der Offenbarung dieser Erfindung ist. Den Transdermalen Systemen wird erfindungsgemäß lediglich mindestens ein µ, κ oder δ Opioid-Antagonist zugegeben.

Dabei kann die Zugabe des µ, κ oder δ Opioid-Antagonisten
a) über eine Beschichtung der bei therapeutischer Nutzung der Haut zugewandten Fläche des Transdermalen Systems mit mindestens einem µ, κ oder δ Opioid-Antagonisten,
b) über eine Beimengung mindestens eines µ, κ oder δ Opioid-Antagonisten zum Wirkstoff,
c) über eine Zugabe mindestens eines µ, κ oder δ Opioid-Antagonisten in die Wirkstoffreservoirschicht bzw. Reservoirschicht,
d) über eine wirkstoffreservoirschicht- bzw. reservoirschicht-seitige Beschichtung der wirkstoffundurchlässigen Rückschicht mit mindestens einem µ, κ oder δ Opioid-Antagonisten oder
e) über eine wirkstoffreservoirschicht- bzw. reservoirschicht-abgewandte Beschichtung der wirkstoffundurchlässigen Rückschicht mit mindestens einem µ, κ oder δ Opioid-Antagonisten
erfolgen. Dabei wird die Nomenklatur und Definition der EP 430 019 bzw. CA 2030178 verwendet, deren Inhalt Teil dieser Beschreibung ist:

Ganz besonders bevorzugt ist die Kombination von Buprenorphin-Base mit Naloxon-Salz.

In einer besonders bevorzugten Ausführungsform des erfindungsgemäßen Transdermalen Systems liegt das auf das Gewicht bezogene Verhältnis der Menge des/der im/auf dem Arzneimittel verwendeten µ, κ oder δ Opioid-Antagonisten zur Menge des/der im Arzneimittel verwendeten µ, κ oder δ Opioid-Agonisten zwischen 1 : 100 und 100 :1, vorzugsweise zwischen 1:20 und 10:1, insbesondere zwischen 1:10 und 1:1, besonders bevorzugt zwischen 1 :10 und 3:10.

In einer anderen bevorzugten Ausführungsform gilt, daß unter der Bedingung, dass das Transdermale System wenigstens 5 Tage in Kontakt mit der Haut bleibt, es eine durchschnittliche Freisetzungsrate von ca. 3 µg/h bis ca. 86 µg/h und einen Anstieg des Plasmaspiegels des Opiod-Agonisten, insbesondere Buprenorphin, prinzipiell erster Ordnung vom Anfang des Dosierungsintervalls bis ca. 72 Stunden nach der Initiierung des Dosierungsintervalls aufrechterhält; und es eine durchschnittliche Freisetzungsrate von ca. 0,3 µg/h bis ca. 9 µg/h und einen Anstieg des Plasmaspiegels des Opiod-Agonisten, insbesondere Buprenorphin, prinzipiell nullter Ordnung von ca. 72 Stunden nach Beginn des Dosierungsintervalls bis zum Ende des Wenigstens-5- Tage-Dosierungsintervalls aufrechterhält, so dass die folgenden durchschnittlichen Plasmakonzentrationen erreicht werden:
eine durchschnittliche Plasmakonzentration von ca. 0,3 bis ca. 113 pg/ml um ca. 6 Stunden nach Beginn des Dosierungsintervalls;
eine durchschnittliche Plasmakonzentration von ca. 3 bis ca. 226 pg/ml um ca. 12 Stunden nach Beginn des Dosierungsintervalls;
eine durchschnittliche Plasmakonzentration von ca. 7 bis ca, 644 pg/ml um ca. 24 Stunden nach Beginn des Dosierungsintervalls;
   eine durchschnittliche Plasmakonzeritration von ca. 13 bis ca. 753 pg/ml um ca. 36 Stunden nach Beginn des Dosierungsintervalls;
   eine durchschnittliche Plasmakonzentration von ca. 13 bis ca. 753 pg/ml um ca. 36 Stunden nach Beginn des Dosierungsintervalls;
   eine durchschnittliche Plasmakonzentration von ca. 16 bis ca. 984 pg/ml um ca. 48 Stunden nach Beginn des Dosierungsintervalls;
   eine durchschnittliche Plasmakonzentration von ca. 20 bis ca. 984 pg/ml um ca. 60 Stunden nach Beginn des Dosierungsintervalls;
   eine durchschnittliche Plasmakonzentration von ca. 21 bis ca. 1052 pg/ml um ca. 72 Stunden nach Beginn des Dosierungsintervalls; und
   eine durchschnittliche Plasmakonzentration von ca. 19 bis ca. 1052 pg/ml um ca. 24 Stunden über wenigstens die nächsten 48 Stunden.

Ein weiterer Gegenstand der Erfindung ist die Verwendung eines erfindungsgemäßen Transdermalen Sytems zur Herstellung eines Arzneimittels zur Behandlung von Schmerz, insbesondere akutem, chronischem, viszeralem, neuropathischem oder Entzündungsschmerz, oder zur Behandlung von vermehrtem Hamdrang bzw. Härninkontinenz.

Der Missbrauch wird bei Umsetzung der hier beschriebenen Erfindung zusätzlich erschwert bis unmöglich gemacht. Ingesamt wird dem buprenorphinhaltigen Pflaster gleichzeitig ein parenteral wirksamer Opioid-Antagonist mit zugesetzt oder das opioidhaltige Pflaster mit einer Opioid-Antagonist-Lösung zusätzlich gecoatet. Dadurch wird beim Versuch, das Buprenorphin aus dem Pflaster herauszulösen, der Antagonist ebenfalls mit herausgelöst. Bei der parenteralen Applikation einer solchen Lösung besetzt der Antagonist aufgrund seiner großen Affinität, beispielsweise zum µ-Opioid-Rezeptor sofort die Rezeptoren und verhindert dadurch die euphorische Wirkung, da eine Rezeptorbindung des Opioids als Agonist dann nicht mehr möglich ist.

Als Opioid-Antagonisten kommen alle geeigneten µ-Rezeptor- oder µ- und k-Rezeptor-Antagonisten in Frage wie z.B. Nalorphin oder Naloxon in Form ihrer Basen oder Salze mit anorganischen (z.B. Chlorid) oder organischen Säuren (z.B. Hydrogencitrat). Bevorzugt wird der Opioid-Antagonist in Form einer praktisch nicht transdermal verfügbaren, aber gut löslichen Verbindung eingesetzt. Dadurch wird das erfindungsgemäße Ziel erreicht, dass transdermal das Buprenorphin als Analgetikum wirken kann, aber bei dem Versuch, das Buprenorphin herauszulösen, der Antagonist mit herausgelöst wird und dieser bei missbräuchlicher parenteraler Applikation der Lösung verhindert, dass das Buprenorphin zur Wirkung kommt.

Die Menge des zugesetzten Opioid-Antagonisten orientiert sich an der Gesamtmenge des in dem Produkt enthaltenen Opioid-Agonisten, bzw. Buprenorphins. Das Verhältnis Opioid-Agonist zu Buprenorphin beträgt bevorzugt 10:1 bis 1:10.

### Beispiele

Die Beispiele dienen der Illustration der vorliegenden Erfindung und bevorzugter Ausführungsformen, sollen aber ihren Schutzumfang nicht beschränken.

### Beispiel 1

### Einarbeiten von Naloxonhydrochlorid in die Wirkstoffmatrix:

1125 g einer 48 Gew.%igen Polyacrylatlösung eines selbstvernetzenden Acrylatcopolymeren aus 2-Ethylhexylacrylat, Vinylacetat, Acrylsäure (Lösemittel: Ethylacetat:Heptan:Isopropanol:Toluol:Acetylacetonat im Verhältnis von 37:26:26:4:1), 100 g Lävulinsäure, 150 g Oleylacetat, 100 g Polyvinylpyrrolidon, 150 g Ethanol, 200 g Ethylacetat, 100 g Buprenorphinbase und 20 g Naloxonhydrochlorid werden homogenisiert. Man rührt etwa zwei Stunden und kontrolliert visuell, ob alle Feststoffe gelöst sind. Man kontrolliert den Verdunstungsverlust durch Zurückwiegen und ergänzt gegebenenfalls den Lösemittelverlust durch Ethylacetat.

Anschließend wird die Mischung so auf eine 420 mm breite, transparente Polyesterfolie aufgetragen, dass das Flächengewicht der getrockneten Kleberschicht bei 80 g/m² liegt. Eine durch Silikonbehandlung wieder ablösbare Polyesterfolie dient als Schutzschicht:

Man entfernt die Lösemittel durch Trocknen mit erwärmter Luft, die über die feuchte Bahn geleitet wird. Durch die Wärmebehandlung verdampfen die Lösemittel. Anschließend deckt man den Kleberfilm mit einer Polyesterfolie 15 µm ab. Mit geeigneten Schneidewerkzeugen stanzt man eine der vorgesehenen Wirkstoffmenge entsprechende Fläche aus und entfernt die zwischen den einzelnen Systemen stehengebliebenen Ränder.

Das so hergestellte Pflaster lehnt sich an das Beispiel 1 der WO 96/19975 oder EP 430 019 A2 an, wobei der gesamte Inhalt dieser Schriften sowie die dort zitierte Literatur Teil der Offenbarung dieser Erfindung ist.

### Beispiel 2:

### Beschichten der Wirkstoffmatrix mit Naloxohhydrochlorid

1139 g einer 48 Gew.%igen Polyacrylatlösung eines selbstvernetzenden Acrylatcopolymeren aus 2-Ethylhexylacrylat, Vinylacetat, Acrylsäure (Lösemittel: Ethylacetat:Heptan:Isopropanol:Toluol:Acetylacetonat im Verhältnis von 37:26:26:4:1), 100 g Lävulinsäure, 150 g Oleylacetat, 100 g Polyvinylpyrrolidon, 150 g Ethanol, 200 g Ethylacetat und 100 g Buprenorphinbase werden homogenisiert. Man rührt etwa zwei Stunden und kontrolliert visuell, ob alle Feststoffe gelöst sind. Man kontrolliert den Verdunstungsverlust durch Zurückwiegen und ergänzt gegebenenfalls den Lösemittelverlust durch Ethylacetat.

Anschließend wird die Mischung so auf eine 420 mm breite, transparente Polyesterfolie aufgetragen, dass das Flächengewicht der getrockneten Kleberschicht bei 80 g/m² liegt. Eine durch Silikonbehandlung wieder ablösbare Polyesterfolie dient als Schutzschicht.

Man entfernt die Lösemittel durch Trocknen mit erwärmter Luft, die über die feuchte Bahn geleitet wird. Durch die Wärmebehandlung verdampfen die Lösemittel. Danach wird die Matrix pro m² mit einer Lösung von 800 mg Naloxonhydrochlorid in 120 ml Methanol beschichtet, beispielsweise durch homogenes aufsprühen, aufspritzen oder aufträufeln, und das Lösemittel durch erneutes Trocknen entfernt.

Anschließend deckt man den Kleberfilm mit einer Polyesterfolie 15 µm ab. Mit geeigneten Schneidewerkzeugen stanzt man eine der vorgesehenen Wirkstoffmenge entsprechende Fläche aus und entfernt die zwischen den einzelnen Systemen stehengebliebenen Ränder.

Das so hergestellte Pflaster lehnt sich an das Beispiel 1 der WO 96/19975 oder EP 430 019 A2 an, wobei der gesamte Inhalt dieser Schriften sowie die dort zitierte Literatur Teil der Offenbarung dieser Erfindung ist.

### Beispiel 3:

### Beschichten der Polyesterfolie mit Naloxonhydrochlorid auf der der Wirkstoffmatrix zugewandten Seite.

1139 g einer 48 Gew.%igen Polyacrylatlösung eines selbstvernetzenden Acrylatcopolymeren aus 2-Ethylhexylacrylat, Vinylacetat, Acrylsäure (Lösemittel: Ethylacetat: Heptan: Isopropanol:Toluol:Acetylacetonat im Verhältnis von 37:26:26:4:1), 100 g Lävulinsäure, 150 g Oleylacetat, 100 g Polyvinylpyrrolidon, 150 g Ethanol, 200 g Ethylacetat und 100 g Buprenorphinbase werden homogenisiert. Man rührt etwa zwei Stunden und kontrolliert visuell, ob alle Feststoffe gelöst sind. Man kontrolliert den Verdunstungsverlust durch Zurückwiegen und ergänzt gegebenenfalls den Lösemittelverlust durch Ethylacetat.

Eine 420 mm breite, transparente Polyesterfolie wird pro m² mit einer Lösung Von 800 mg Naloxonhydrochlorid in 120 ml Methanol beschichttet und das Lösemittel durch Trocknen entfernt. Anschließend wird die oben beschriebene Mischung so auf die beschichtete Seite der beschichteten Polyesterfolie aufgetragen, dass das Flächengewicht der getrockneten Kleberschicht bei 80 g/m² liegt. Eine durch Silikonbehandlung wieder ablösbare Polyesterfolie dient als Schutzschicht.

Man entfernt die Lösemittel durch Trocknen mit erwärmter Luft, die über die feuchte Bahn geleitet wird. Durch die Wärmebehandlung verdampfen die Lösemittel.

Anschließend deckt man den Kleberfilm mit einer 15 µm dicken Polyesterfolie ab. Mit geeigneten Schneidewerkzeugen stanzt man eine der vorgesehenen Wirkstoffmenge entsprechende Fläche aus und entfernt die zwischen den einzelnen Systemen stehengebliebenen Ränder.

Das so hergestellte Pflaster lehnt sich an das Beispiel 1 der WO 96/19975 oder EP 430 019 A2 an, wobei der gesamte Inhalt dieser Schriften sowie die dort zitierte Literatur Teil der Offenbarung dieser Erfindung ist.

### Beispiel 4:

### Beschichten der Polyesterfolie mit Naloxonhydrochlorid auf der der Wirkstoffmatrix abgewandten Seite.

1139 g einer 48 Gew.%igen Polyacrylatlösung eines selbstvernetzenden Acrylatcopolymeren aus 2-Ethylhexylacrylat, Vinylacetat, Acrylsäure (Lösemittel: Ethylacetat:Heptan:Isopropanol:Toluol:Acetylacetonat im Verhältnis von 37:26:26:4:1), 100 g Lävulinsäure, 150 g Oleylacetat, 100 g Polyvinylpyrrolidon, 150 g Ethanol, 200 g Ethylacetat und 100 g Buprenorphinbase werden homogenisiert. Man rührt etwa zwei Stunden und kontrolliert visuell, ob alle Feststoffe gelöst sind. Man kontrolliert den Verdunstungsverlust durch Zurückwiegen und ergänzt gegebenenfalls den Lösemittelverlust durch Ethylacetat.

Eine 420 mm breite, transparente Polyesterfolie wird pro m² mit einer Lösung von 800 mg Naloxonhydrochlorid in 120 ml Methanol beschichttet und das Lösemittel durch Trocknen entfernt. Dann wird die Folie so umgedreht, dass die unbeschichtete Seite nach oben zeigt. Anschließend wird die oben beschriebene Mischung so auf die unbeschichtete Seite der beschichteten Polyesterfolie aufgetragen, dass das Flächengewicht der getrockneten Kleberschicht bei 80 g/m² liegt. Eine durch Silikonbehandlung wieder ablösbare Polyesterfolie dient als Schutzschicht.

Man entfernt die Lösemittel durch Trocknen mit erwärmter Luft, die über die feuchte Bahn geleitet wird. Durch die Wärmebehandlung verdampfen die Lösemittel.

Anschließend deckt man den Kleberfilm mit einer 15 µm dicken Polyesterfolie ab. Mit geeigneten Schneidewerkzeugen stanzt man eine der vorgesehenen Wirkstoffmenge entsprechende Fläche aus und entfernt die zwischen den einzelnen Systemen stehengebliebenen Ränder.

Das so hergestellte Pflaster lehnt sich an das Beispiel 1 der WO 96/19975 bzw. US 6,246,980 oder EP 430 019 A2 bzw. CA 2030178 an, wobei der gesamte Inhalt dieser Schriften sowie die dort zitierte Literatur Teil der Offenbarung dieser Erfindung ist.

### Beispiel 5:

### Permeationsversuche durch Stratum corneum

Permeation von NaloxonHCl neben Buprenorphin(Base) aus einem Transdermalen System hergestellt nach Beispielen 1 bis 4.

### 1. Aufbau Penetrationsmodell:

Franz-Zelle mit ca. 100 ml Akzeptorvolumen.
Zuerst wird ein geeigneter Magnetfisch in den Akzeptorraum gegeben.
Die Kontaktflächen zum Fixieren der Permeationsbarriere (hier: Stratum comeum) werden mit Silikonpaste (Schlifffett) bestrichen und
akzeptorseitig als Unterstützung ein Polycarbonatfilter mit 5µm Porenweite mittig aufgebracht (Millipore TMTP025). Auf dieser Unterstützungsmembran wird das ausgestanzte, unbeschädigte und vor Einsatz gewässerte Stück Stratum corneum aufgelegt und und darüber das zu prüfende Pflasterstück als Donor aufgebracht. Danach wird das Oberteil der Franz-Zelle aufgesetzt und zum Unterteil mit einer Klammer gesichert.
Daraufhin wird das entgaste, ca. 34°C warme Akzeptormedium blasenfrei über das Steigrohr eingefüllt. Die Füllmenge an Akzeptor ist so gewählt, daß ein vollständiger Kontakt des Akzeptors mit der Permeationsbarriere gewährleistet ist. Der Flüssigkeitsspiegel im Steigrohr liegt immer über der Ebene der Permeationsbarriere.

Zur Beschwerung des donorseitigen Pflasters wird ein HPLC-vial auf dessen Oberfläche gestellt und der donorseitige Stutzen mit Parafilm oder einem anderen Verschlußsystem verschlossen.

Zum Start des Versuches wird die vorbereitete Franz-Zelle so in ein auf 34°C thermostatisiertes Wasserbad gehängt, dass die Badflüssigkeit praktisch vollständig das Akzeptormedium erreicht, aber keine Gefahr besteht, dass es an der Trennstelle Donor/ Akzeptorbereich in die Franz-Zelle gelangt.
Das Bad mit der Franz-Zelle wird so auf einem Magnetrührer platziert, dass permanent sowohl Badfüssigkeit und Akzeptormedium über Magnetfische gerührt werden.

### 2. Donor

Als Donor dient das entsprechend zugeschnittene Pflaster

### 3. Akzeptor

Als Akzeptor dient eine isotonische Phosphatpufferlösung, die in 1 l wässriger Lösung folgende Salze gelöst enthält:
1,225 g Kaliumdihydrogenphosphat, wasserfrei
4,26 g Dinatriumhydrogenphosphat, wasserfrei
6,00 g Natriumchlorid

Probenahme für analytische Zwecke: ca. 10 ml

### 4. Permeationsbarriere:

Als Permeationsbarriere dient das Stratum corneum (S.c.).

Lagerung seit 9.4.2002 im Exsikkator mit Trockenmittel bei RT. Vor Einsatz wir das S.c. mikroskopisch begutachtet, entsprechende Areale als Barriere ausgewählt und ausgeschnitten.

### 5. Probenahmen

Probenahmemengen:
jeweils zu jedem Probenahmezeitpunkt werden 2x 200 µl mit der Varipette entnommen und jeweils die entnommene Menge durch Isotonische Phosphatpufferlösung ersetzt.
Die Probenahmen erfolgen mit der Varipette zu jedem Probenahmezeitpunkt und bei jeder Zugabe von Pufferlösung wird eine neue Pipettenspitze verwendet.
Die Einstellung der Varipette erfolgt auf einen Wert, der erforderlich ist, um durchschnittlich 200 µl abzugeben (203 µl).
Alle entnommenen Proben werden zusätzlich gewogen und dokumentiert. Die entnommenen Proben werden in HPLC-vials gefüllt, zugebördelt, etikettiert und eingefroren.

### 6. Probenahmezeiten:

Von jeder Serie werden nach 0,5; 1,0; 1,5; 2; 4; 6; 24; 48 Stunden jeweils 2 Proben zu 200 µl gezogen.

## Patentansprüche

1. Transdermales System bzw. Trandermales Therapeutisches System enthaltend als Wirkstoff mindestens Buprenorphin in Form der Base oder eines Salzes, **dadurch gekennzeichnet, dass** es mindestens einen µ, κ oder δ Opioidrezeptor-Antagonisten; gegebenenfalls in Form seiner Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form der Säuren oder Basen oder in Form der Salze, insbesondere der physiologisch verträglichen Salze, oder in Form der Solvate, insbesondere der Hydrate; enthält.

2. Transdermales System gemäß Anspruch 1, **dadurch gekennzeichnet, dass** der µ, κ oder δ Opioidrezeptor-Antagonist ein µ Opioidrezeptor-Antagonist bzw. Morphinantagonist ist, vorzugsweise Levallorphan, Naltrexon, Nalorphin oder Naloxon, insbesondere Naloxon.

3. Transdermales System gemäß Anspruch 2, **dadurch gekennzeichnet, dass** Levallorphan, Naltrexon, Nalorphin oder Naloxon in Form ihrer Basen oder in Form ihrer Salze, insbesondere der physiologisch verträglichen Salze, vorzugsweise der mit anorganischen oder organischen Säuren gebildeten Salze, insbesondere als Chlorid- Bromid- oder Hydrogencitratsalz vorliegt.

4. Transdermales System gemäß einem der Ansprüche 1 bis 3, **dadurch gekennzeichnet, dass** das Buprenorphin als Base vorliegt.

5. Transdermales System gemäß einem der Ansprüche 1 bis 4, **dadurch gekennzeichnet, dass** der µ, κ oder δ Opioid-Antagonist
a) über eine Beschichtung der bei therapeutischer Nutzung der Haut zugewandten Fläche des Transdermalen Systems mit mindestens einem µ, κ oder δ Opioid-Antagonisten,
b) über eine Beimengung mindestens eines µ, κ oder δ Opioid-Antagonisten zum Wirkstoff,
c) über eine Zugabe mindestens eines µ, κ oder δ Opioid-Antagonisten in die Wirkstoffreservoirschicht bzw. Reservoirschicht,
d) über eine wirkstoffreservoirschicht- bzw. reservoirschicht-seitige Beschichtung der wirkstoffundurchlässigen Rückschicht mit mindestens einem µ, κ oder δ Opioid-Antagonisten und/oder
e) über eine wirkstoffreservoirschicht- bzw. reservoirschicht-abgewandte Beschichtung der wirkstoffundurchlässigen Rückschicht mit mindestens einem µ, κ oder δ Opioid-Antagonisten
zugegeben wird.

6. Transdermales System gemäß einem der Anspruch 1 bis 5 , **dadurch gekennzeichnet, dass** das auf das Gewicht bezogenen Verhältnis der Menge des/der im/auf dem Arzneimittel verwendeten µ, κ oder δ Opioid-Antagonisten zur Menge des Buprenorphins zwischen 1 : 100 und 10 :1, vorzugsweise zwischen 1:20 und 5:1, insbesondere zwischen 1:10 und 1:1, besonders bevorzugt zwischen 1 :10 und 3:10 liegt.

7. Transdermales System gemäß einem der Ansprüche 1 bis 6, **dadurch gekennzeichnet, daß** unter der Bedingung, dass das Transdermale System wenigstens 5 Tage in Kontakt mit der Haut bleibt, es eine durchschnittliche Freisetzungsrate von ca. 3 µg/h bis ca. 86 µg/h und einen Anstieg des Plasmaspiegels des Buprenorphins, prinzipiell erster Ordnung vom Anfang des Dosierungsintervalls bis ca. 72 Stunden nach der Initiierung des Dosierungsintervalls aufrechterhält; und es eine durchschnittliche Freisetzungsrate von ca. 0,3 µg/h bis ca. 9 µg/h und einen Anstieg des Plasmaspiegels des Opiod-Agonisten, insbesondere Buprenorphin, prinzipiell nullter Ordnung von ca. 72 Stunden nach Beginn des Dosierungsintervalls bis zum Ende des Wenigstens-5-Tage-Dosierungsintervalls aufrechterhält, so dass die folgenden durchschnittlichen Plasmakonzentrationen erreicht werden:
eine durchschnittliche Plasmakonzentration von ca. 0,3 bis ca. 113 pg/ml um ca. 6 Stunden nach Beginn des Dosierungsintervalls;
eine durchschnittliche Plasmakonzentration von ca. 3 bis ca. 226 pg/ml um ca. 12 Stunden nach Beginn des Dosierungsintervalls;
eine durchschnittliche Plasmakonzentration von ca. 7 bis ca. 644 pg/ml um ca. 24 Stunden nach Beginn des Dosierungsintervalls;
eine durchschnittliche Plasmakonzentration von ca. 13 bis ca. 753 pg/ml um ca. 36 Stunden nach Beginn des Dosierungsintervalls;
eine durchschnittliche Plasmakonzentration von ca. 13 bis ca. 753 pg/ml um ca. 36 Stunden nach Beginn des Dosierungsintervalls;
eine durchschnittliche Plasmakonzentration von ca. 16 bis ca. 984 pg/ml um ca. 48 Stunden nach Beginn des Dosierungsintervalls;
eine durchschnittliche Plasmakonzentration von ca. 20 bis ca. 984 pg/ml um ca. 60 Stunden nach Beginn des Dosierungsintervalls;
eine durchschnittliche Plasmakonzentration von ca. 21 bis ca. 1052 pg/ml um ca. 72 Stunden nach Beginn des Dosierungsintervalls; und
eine durchschnittliche Plasmakonzentration von ca. 19 bis ca. 1052 pg/ml um ca. 24 Stunden über wenigstens die nächsten 48 Stunden.

8. Verwendung eines Transdermales Systems gemäß einem der Anspruche 1 bis 7 zur Herstellung eines Arzneimittels zur Behandlung von Schmerz, insbesondere akutem, chronischem, viszeralem, neuropathischem oder Entzündungsschmerz, oder zur Behandlung von vermehrtem Hamdrang bzw. Harninkontinenz.

## Claims

1. Transdermal system or transdermal therapeutic system containing as the active ingredient at least buprenorphine in the form of the base or of a salt, **characterised in that** it contains at least a µ, κ or δ opioid receptor antagonist; optionally in the form of its racemates, the pure stereoisomers, in particular enantiomers or diastereomers, or in the form of blends of stereoisomers, in particular the enantiomers or diastereomers, in any mixing ratio; in the form of the acids or bases or in the form of the salts, in particular the physiologically acceptable salts, or in the form of the solvates, in particular the hydrates.

2. Transdermal system according to claim 1, **characterised in that** the µ, κ oder δ opioid receptor antagonist is a µ opioid receptor antagonist or morphine antagonist, preferably levallorphan, naltrexone, nalorphine or naloxone, in particular naloxone.

3. Transdermal system according to claim 2, **characterised in that** levallorphan, naltrexone, nalorphine or naloxone is in the form of its bases or in the form of its salts, in particular the physiologically acceptable salts, preferably the salts formed with inorganic or organic acids, in particular in the form of the chloride-bromide salt or hydrogen citrate salt.

4. Transdermal system according to any one of claims 1 to 3, **characterised in that** the buprenorphine is in the form of a base.

5. Transdermal system according to any one of claims 1 to 4, **characterised in that** the µ, κ or δ opioid antagonist is added
a) by coating the surface of the transdermal system facing the skin with at least a µ, κ or δ opioid antagonist during therapeutic use,
b) by incorporating at least a µ, κ or δ opioid antagonist in the active ingredient,
c) by adding at least a µ, κ or δ opioid antagonist to the active ingredient reservoir layer or reservoir layer,
d) by coating the backing layer, which is impermeable to active ingredient, with at least a µ, κ or δ opioid antagonist, on the side facing the active ingredient reservoir layer or reservoir layer and/or
e) by coating the backing layer, which is impermeable to active ingredient, with at least a µ, κ or δ opioid antagonist, on the side remote from the active ingredient reservoir layer or reservoir layer.

6. Transdermal system according to any one of claims 1 to 5, **characterised in that** the ratio by weight of the amount of µ, κ or δ opioid antagonists used in the pharmaceutical composition to the amount of buprenorphine is between 1:100 and 10:1, preferably between 1:20 and 5:1, in particular between 1:10 and 1:1, more preferably between 1:10 and 3:10.

7. Transdermal system according to any one of claims 1 to 6, **characterised in that**, providing that the transdermal system remains in contact with the skin for at least 5 days, it maintains an average release rate from about 3 µg/h to about 86 µg/h and an increase in the plasma level of the buprenorphine, basically of the first order from commencement of the dosing interval to about 72 hours after initiation of the dosing interval; and maintains an average release rate from about 0.3 µg/h to about 9 µg/h and an increase in the plasma level of the opioid agonist, in particular buprenorphine, basically of the zero-th order from about 72 hours after commencement of the dosing interval to the end of the at least 5-day dosing interval, so the following average plasma concentrations are achieved:
an average plasma concentration from about 0.3 to about 113 pg/ml about 6 hours after commencement of the dosing interval;
an average plasma concentration from about 3 to about 226 pg/ml about 12 hours after commencement of the dosing interval; an average plasma concentration from about 7 to about 644 pg/ml about 24 hours after commencement of the dosing interval;
an average plasma concentration from about 13 to about 753 pg/ml about 36 hours after commencement of the dosing interval;
an average plasma concentration from about 16 to about 984 pg/ml about 48 hours after commencement of the dosing interval;
an average plasma concentration from about 20 to about 984 pg/ml about 60 hours after commencement of the dosing interval;
an average plasma concentration from about 21 to about 1052 pg/ml about 72 hours after commencement of the dosing interval; and
an average plasma concentration from about 19 to about 1052 pg/ml for about 24 hours over at least the next 48 hours.

8. Use of a transdermal system according to any one of claims 1 to 7 for producing a pharmaceutical composition for the treatment of pain, in particular acute, chronic, visceral or neuropathic pain or pain caused by inflammation, or for the treatment of an increased urge to urinate or urinary incontinence.

## Revendications

1. Système transdermique ou système thérapeutique transdermique, contenant comme substance active au moins de la buprénorphine sous forme de la base ou d'un sel, **caractérisé en ce qu'**il contient au moins un antagoniste des récepteurs opioïdes µ, κ ou δ ; éventuellement sous forme de ses racémates, des stéréo-isomères purs, en particulier des énantiomères ou des diastéréoisomères, ou sous forme de mélanges des stéréo-isomères, en particulier des énantiomères ou des diastéréoisomères, dans un rapport de mélange quelconque ; sous forme des acides ou des bases ou sous forme des sels, en particulier des sels physiologiquement acceptables, ou sous forme des produits de solvatation, en particulier des hydrates.

2. Système transdermique suivant la revendication 1, **caractérisé en ce que** l'antagoniste des récepteurs opioïdes µ, κ ou δ est un antagoniste des récepteurs opioïdes µ ou un antagoniste de la morphine, avantageusement le lévallorphane, la naltrexone, la nalorphine ou la naloxone, en particulier la naloxone.

3. Système transdermique suivant la revendication 2, **caractérisé en ce que** le lévallorphane, la naltrexone, la nalorphine ou la naloxone se présentent sous forme de leurs bases ou sous forme de leurs sels, en particulier des sels physiologiquement acceptables, avantageusement des sels formés avec des acides inorganiques ou organiques, en particulier sous forme de chlorure, de bromure ou d'hydrogénocitrate.

4. Système transdermique suivant l'une des revendications 1 à 3, **caractérisé en ce que** la buprénorphine est présente sous forme de base.

5. Système transdermique suivant l'une des revendications 1 à 4, **caractérisé en ce que** l'antagoniste des récepteurs opioïdes µ, κ ou δ est ajouté
a) par revêtement de la surface du système transdermique tournée vers la peau lors de l'application thérapeutique d'au moins un antagoniste des récepteurs opioïdes µ, κ ou δ,
b) par l'addition d'au moins un antagoniste des récepteurs opioïdes µ, κ ou δ à la substance active,
c) par l'addition d'au moins un antagoniste des récepteurs opioïdes µ, κ ou δ dans la couche réservoir de substance active ou couche réservoir,
d) par revêtement, du côté couche réservoir de substance active ou couche réservoir, de la couche postérieure imperméable à la substance active, d'au moins un antagoniste des récepteurs opioïdes µ, κ ou δ, et/ou
e) par revêtement, opposé à la couche réservoir de substance active ou couche réservoir, de la couche postérieure imperméable à la substance active d'au moins un antagoniste des récepteurs opioïdes µ, κ ou δ.

6. Système transdermique suivant l'une des revendications 1 à 5, **caractérisé en ce que** le rapport en poids de la quantité du ou des antagonistes des récepteurs opioïdes µ, κ ou δ utilisés dans ou sur le médicament à la quantité de buprénorphine se situe entre 1:100 et 10:1, avantageusement entre 1:20 et 5:1, en particulier entre 1:10 et 1:1, très avantageusement entre 1:10 et 3:10.

7. Système transdermique suivant l'une des revendications 1 à 6, **caractérisé en ce que**, sous réserve que le système transdermique reste au contact de la peau pendant au moins 5 jours, on considère qu'une vitesse moyenne de libération d'environ 3 µg/h à environ 86 µg/h et une élévation du taux plasmatique de buprénorphine, en principe d'ordre un, sont maintenues depuis le début de l'intervalle de dosage jusqu'à environ 72 heures après l'entrée en action de l'intervalle de dosage ; et qu'une vitesse moyenne de libération d'environ 0,3 µg/h à environ 9 µg/h et une élévation du taux plasmatique de l'agoniste des récepteurs opioïdes, en particulier de buprénorphine, en principe d'ordre nul, sont maintenues depuis environ 72 heures après le début de l'intervalle de dosage jusqu'à la fin de l'intervalle de dosage d'au moins 5 jours, si bien que les concentrations plasmatiques moyennes suivantes sont atteintes :
une concentration plasmatique moyenne d'environ 0,3 à environ 113 pg/ml environ 6 heures après le début de l'intervalle de dosage ;
une concentration plasmatique moyenne d'environ 3 à environ 226 pg/ml environ 12 heures après le début de l'intervalle de dosage ;
une concentration plasmatique moyenne d'environ 7 à environ 644 pg/ml environ 24 heures après le début de l'intervalle de dosage ;
une concentration plasmatique moyenne d'environ 13 à environ 753 pg/ml environ 36 heures après le début de l'intervalle de dosage ;
une concentration plasmatique moyenne d'environ 13 à environ 753 pg/ml environ 36 heures après le début de l'intervalle de dosage ;
une concentration plasmatique moyenne d'environ 16 à environ 984 pg/ml environ 48 heures après le début de l'intervalle de dosage ;
une concentration plasmatique moyenne d'environ 20 à environ 984 pg/ml environ 60 heures après le début de l'intervalle de dosage ;
une concentration plasmatique moyenne d'environ 21 à environ 1052 pg/ml environ 72 heures après le début de l'intervalle de dosage ; et
une concentration plasmatique moyenne d'environ 19 à environ 1052 pg/ml environ 24 heures au-delà d'au moins les 48 heures suivantes.

8. Utilisation d'un système transdermique suivant l'une des revendications 1 à 7, pour la préparation d'un médicament destiné au traitement de la douleur, en particulier d'une douleur aiguë, chronique, viscérale, neuropathique ou liée à une inflammation, ou destiné au traitement du besoin répété d'uriner ou de l'incontinence d'urine.
